# EUROPEAN PATENT APPLICATION

(11) **EP 4 684 744 A2**
(43) Date of publication of application: **28.01.2026**
(21) Application number: 25189855.7
(22) Date of filing: 16.07.2025
(51) Int. Cl.: A61B 17/17, A61B 17/80

(54) **INTERNAL BEAM PLATES AND ASSOCIATED INSTRUMENTATION FOR PERFORMING SURGICAL METHODS**

(30) Priority: 25.07.2024 US 202463675378 P
(71) Applicant: Arthrex, Inc, Naples, FL 34108-1945 (US)
(72) Inventor: MORGAN, Alyssa, Naples, 34109 (US); POWELL, Chris, Naples, 34109 (US); RICCIARDI, Giovanni, 80339 Munich (DE); HASSELMAN, Carl, Oakmont, 15139 (US)
(74) Representative: Marks & Clerk LLP

(57) **Abstract**

Internal beam plates and associated instrumentation may be utilized for performing arthrodesis procedures, bone fracture procedures, osteotomy procedures, etc. Exemplary surgical methods that may be performed using the internal beam plates and associated instrumentation include, but are not limited to, Lapidus bunionectomy procedures, metatarsophalangeal (MTP) procedures, chevron procedures, fracture repair procedures, etc. The exemplary internal beam plates incorporate an integral beam that is configured to increase strength and stiffness in two planes across a fusion site. The exemplary cutting guides are configured to allow surgeons to prepare bones for receiving the beam of the internal beam plate.

## Description

### CROSS REFERENCE TO RELATED APPLICATIONS

This application claims the benefit of United States Provisional Application No. 63/675,378, which was filed on July 25, 2024 and is incorporated herein by reference in its entirety.

### BACKGROUND

This disclosure relates to the field of surgery, and more particularly to internal beam bone plates and their associated instrumentation and surgical methods.

A variety of surgical devices are commonly used to treat bone abnormalities, such as bunions and fractures. For example, bone plates are commonly employed during orthopedic surgeries to stabilize, fuse, and/or align bones or bone fragments in order to restore functionality to a joint.

### SUMMARY

This disclosure is directed to internal beam plates and associated instrumentation for performing arthrodesis procedures (i.e., bone fusion procedures), bone fracture procedures, osteotomy procedures, etc.

An exemplary internal beam plate may include, *inter alia,* a plate body and a beam. The plate body extends along a longitudinal axis between a first portion and a second portion and includes a bone contacting surface and an outer surface opposed to the bone contacting surface. The beam protrudes outwardly from the bone contacting surface. A cut-out section is formed in the beam.

An exemplary cutting guide may include, *inter alia,* a base extending along a longitudinal centerline axis between a first portion and a second portion and including a bone contacting surface and an outer surface opposed to the bone contacting surface, a raised housing protruding outward from the outer surface of the base, and a longitudinal cutting slot formed through the raised housing and the base and extending along the longitudinal centerline axis.

An exemplary surgical method may include, *inter alia,* fixating a cutting guide to a first bone and a second bone, inserting a surgical cutting device through a longitudinal cutting slot of the cutting guide, a cutting path of the surgical cutting device being delimited within the longitudinal cutting slot by a raised housing of the cutting guide, preparing a slot within the first bone and the second bone, removing the cutting guide, positioning a beam of an internal beam plate within the slot, and fixating the internal beam plate to both the first bone and the second bone.

### BRIEF DESCRIPTION OF THE DRAWINGS

Figure 1 illustrates a foot of the human musculoskeletal system. The foot includes a bone abnormality.
Figure 2 is a perspective view of an exemplary internal beam plate for correcting a bone abnormality.
Figure 3 is a top view of the internal beam plate of Figure 2.
Figure 4 is a bottom view of the internal beam plate of Figure 2.
Figure 5 is a side view of the internal beam plate of Figure 2.
Figure 6 is an end view of the internal beam plate of Figure 2.
Figures 7A, 7B, and 7C illustrate another exemplary internal beam plate.
Figure 8 is a perspective view of an exemplary cutting guide for preparing a joint for receiving an internal beam plate.
Figure 9 is a top view of the cutting guide of Figure 8.
Figure 10 is a bottom view of the cutting guide of Figure 8.
Figures 11A, 11B, and 11C illustrate another exemplary cutting guide.
Figures 12, 13, 14, 15, 16, 17, 18, and 19 schematically illustrate an exemplary surgical method for performing an arthrodesis procedure that involves the implantation of an internal beam plate.

### DETAILED DESCRIPTION

This disclosure is directed to internal beam plates and associated instrumentation for performing arthrodesis procedures, bone fracture procedures, osteotomy procedures, etc. Exemplary surgical methods that may be performed using the internal beam plates and associated instrumentation described herein include, but are not limited to, Lapidus bunionectomy procedures, metatarsophalangeal (MTP) procedures, chevron procedures, fracture repair procedures, etc. The exemplary internal beam plates incorporate an integral beam that is configured to increase the strength and stiffness in two planes across a fusion site. The exemplary cutting guides are configured to allow surgeons to prepare and resect bones for receiving the beam of the internal beam plate. These and other features of this disclosure are described in further detail below.

An exemplary internal beam plate may include, *inter alia,* a plate body and a beam. The plate body extends along a longitudinal axis between a first portion and a second portion and includes a bone contacting surface and an outer surface opposed to the bone contacting surface. The beam protrudes outwardly from the bone contacting surface. A cut-out section is formed in the beam.

In a further embodiment, the bone contacting surface includes a curvature for conforming to a contour of a bone.

In a further embodiment, the plate body and the beam are integrated to establish a single-piece structure that excludes mechanical attachments for connecting the plate body and the beam together.

In a further embodiment, the beam protrudes away from the bone contacting surface along a beam axis that is substantially perpendicular to the longitudinal centerline axis.

In a further embodiment, a distance the beam protrudes from the bone contacting surface is smaller than a first length of the beam and a second length of the plate body.

In a further embodiment, the first length is less than the second length.

In a further embodiment, a first set of openings is formed through the first portion of the plate body, and a second set of openings is formed through the second portion of the plate body.

In a further embodiment, the cut-out section is flanked by a first beam section and a second beam section of the beam.

In a further embodiment, a bridging section of the beam connects between the first beam section and the second beam section.

In a further embodiment, the first beam section and the second beam section each extend a first distance from the bone contacting surface of the plate body, and the bridging section extends a second, lesser distance from the bone contacting surface of the plate body.

An exemplary cutting guide may include, *inter alia,* a base extending along a longitudinal centerline axis between a first portion and a second portion and including a bone contacting surface and an outer surface opposed to the bone contacting surface, a raised housing protruding outward from the outer surface of the base, and a longitudinal cutting slot formed through the raised housing and the base and extending along the longitudinal centerline axis.

In a further embodiment, the base includes an intermediate portion that connects between the first portion and the second portion, and the raised housing protrudes outwardly from the outer surface at the intermediate portion.

In a further embodiment, an alignment feature is provided at an exterior surface of the raised housing.

In a further embodiment, at least one aperture is formed through each of the first portion and the second portion of the base.

In a further embodiment, the raised housing is ovoid-shaped.

An exemplary surgical method may include, *inter alia,* fixating a cutting guide to a first bone and a second bone, inserting a surgical cutting device through a longitudinal cutting slot of the cutting guide, a cutting path of the surgical cutting device being delimited within the longitudinal cutting slot by a raised housing of the cutting guide, preparing a slot within the first bone and the second bone, removing the cutting guide, positioning a beam of an internal beam plate within the slot, and fixating the internal beam plate to both the first bone and the second bone.

In a further embodiment, fixating the cutting guide includes inserting at least one K-wire through a base of the cutting guide and into the first bone, and inserting at least one additional K-wire through the base and into the second bone.

In a further embodiment, preparing the slot includes moving the surgical cutting device in a first direction within the longitudinal cutting slot, and moving the surgical cutting device in a second opposite direction within the longitudinal cutting slot.

In a further embodiment, a first portion of the slot is formed in the first bone, and a second portion of the slot is formed in the second bone.

In a further embodiment, positioning the beam of the internal beam plate within the slot includes positioning a first beam section of the beam within the first portion of the slot, and positioning a second beam section of the beam within the second portion of the slot.

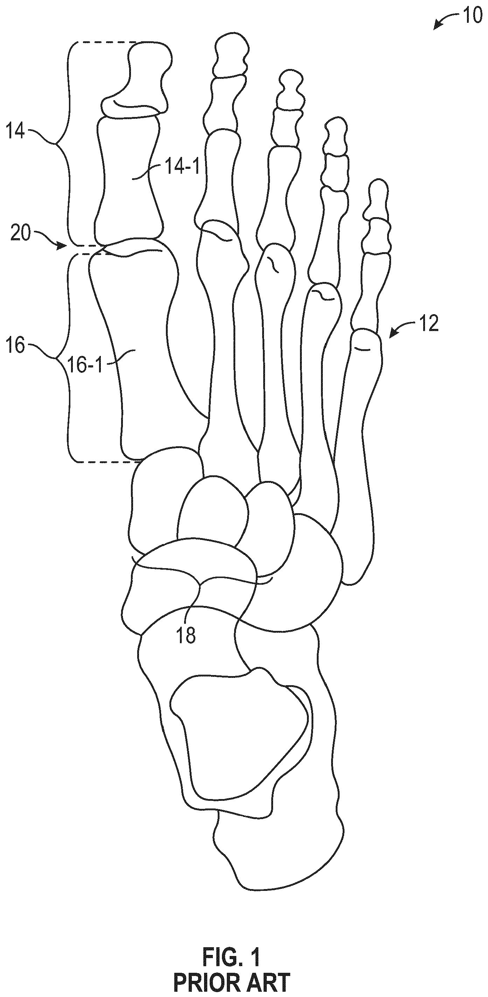

Figure 1 schematically illustrates select portions of a foot 10 of the human musculoskeletal system. A forefoot 12 of the foot 10 is specifically shown. The forefoot 12 includes multiples phalanges 14 (i.e., toes), multiples metatarsals 16 located proximal to the phalanges 14, and a trio of cuneiforms 18 (i.e., medial, middle, and lateral cuneiforms) located proximal to the metatarsals 16. The cuneiforms 18 form portions of a set of tarsal bones of the foot 10.

As schematically illustrated, the foot 10 includes a bone abnormality 20. In an embodiment, the bone abnormality 20 is a hallux valgus abnormality (also referred to as a bunion abnormality) in which there is a medial deviation of a first metatarsal 16-1 and a lateral deviation of a first phalange 14-1. If not corrected, the bone abnormality 20 can lead to pain, arthritis, and/or ailments.

Exemplary internal beam plates and associated instrumentation designed for repairing the bone abnormality 20 are detailed herein. Although the various teachings of this disclosure are detailed with reference to a bunion abnormality occurring at the first metatarsophalangeal (MTP) joint of the foot 10, the exemplary internal beam plates and instrumentation described herein may be used to repair other bone abnormalities, including but not limited to fractures of the foot, ankle, hand, wrist, etc.

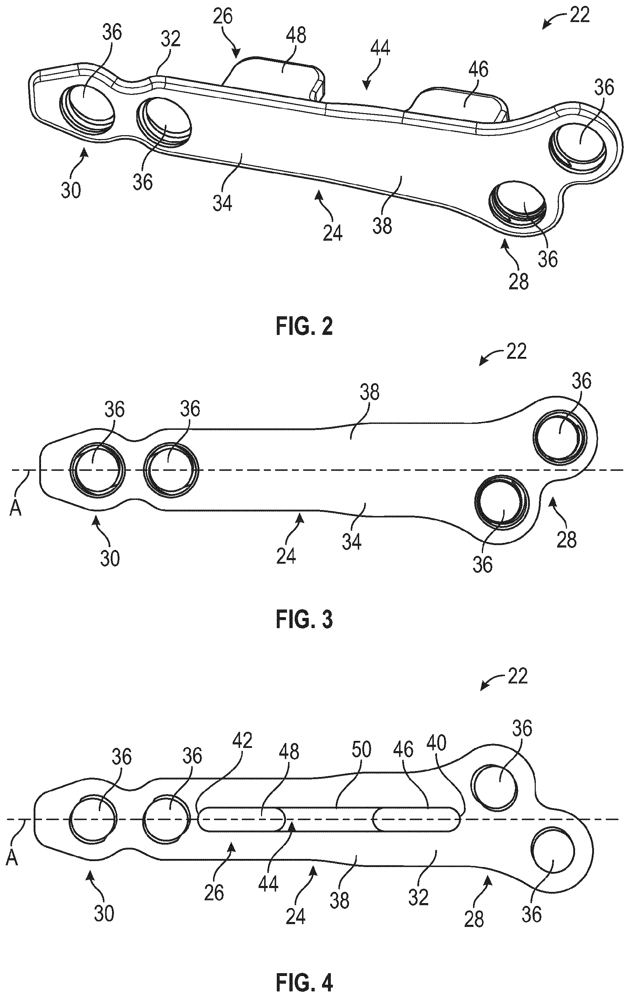

Figures 2-6 illustrate an exemplary internal beam plate 22 for correcting bone abnormalities, such as the bone abnormality 20 of Figure 1, for example. The internal beam plate 22 is a bone plate that may include a plate body 24 and a beam 26 that extends outwardly from the plate body 24. In an embodiment, the plate body 24 and the beam 26 are integrated with one another to establish a single-piece structure. Stated another way, the internal beam plate 22 may be a monolithic bone plate without any mechanical attachments for connecting the plate body 24 and the beam 26 together. In an embodiment, the beam 26 is a machined portion of the internal beam plate 22.

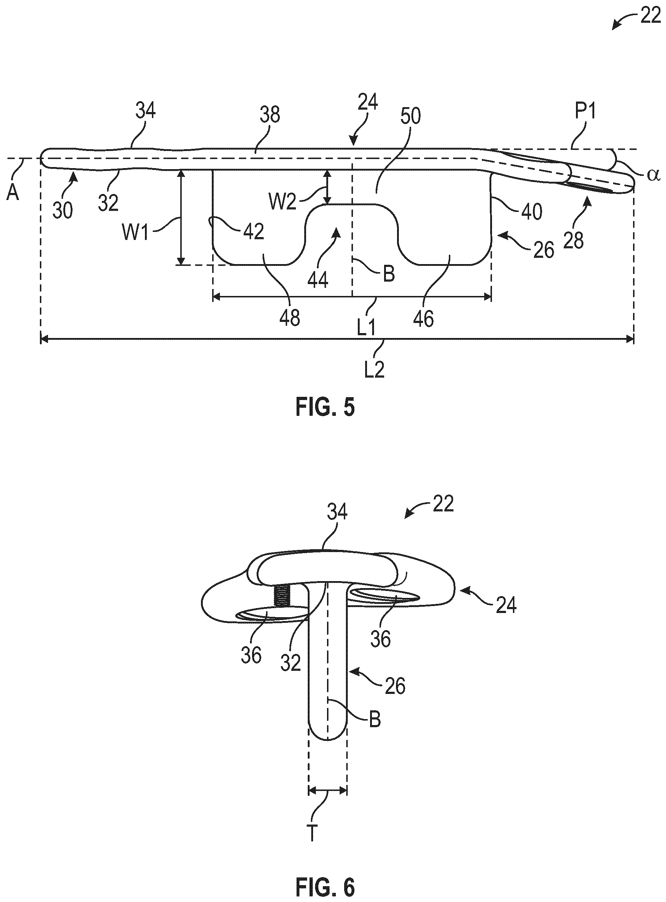

The plate body 24 extends along a longitudinal centerline axis A between a first or proximal portion 28 and a second or distal portion 30. An intermediate portion 38 extends between the first portion 28 and the second portion 30. The plate body 24 may include any size and shape. The plate body 24 may additionally include a bone contacting surface 32 and an outer surface 34 on an opposite side of the plate body 24 from the bone contacting surface 32. The bone contacting surface 32 may include a slight curvature (see, e.g., Figure 6) for conforming to the anatomical contour of one or more bones.

The first portion 28 may be angled relative to the intermediate portion 38 of the plate body 24 in order to conform to the natural curvature of the bone anatomy. For example, the first portion 28 may extend at an angle α relative to a plane P1 (see Figure 5) that extends through the outer surface 34 of the plate body 24 to establish the angled relationship between the first portion 28 and the intermediate portion 38. In an embodiment, the angle α is about 10 degrees (+/- 2.5 degrees, for example). However, other angles may be suitable within the scope of this disclosure. In this disclosure, the term "about" means that the expressed quantities or ranges need not be exact but may be approximated and/or larger or smaller, reflecting acceptable tolerances, conversion factors, measurement error, etc.

The beam 26 may protrude outwardly from the bone contacting surface 32 in an opposite direction from the outer surface 34 of the plate body 24. The beam 26 may protrude from the intermediate portion 38 of the plate body 24 that connects between the first and second portions 28, 30. In an embodiment, the beam 26 protrudes away from the plate body 24 along a beam axis B (see Figure 5) that is substantially perpendicular to the longitudinal centerline axis A. As further discussed below, the beam 26 may be accommodated within one or more pre-formed bone slots for increasing the strength, rigidity, and stability in two planes across a fusion site.

The beam 26 includes a length L1 (see Figure 5), a width W1 (see Figure 5), and a thickness T1 (see Figure 6). The length L1 extends in parallel with the longitudinal centerline axis A of the plate body 24 from one lengthwise end 40 to an opposite lengthwise end 42 of the beam 26. The lengthwise ends 40, 42 may be flat or chamfered.

In an embodiment, the length L1 of the beam 26 is shorter than a length L2 of the plate body 24. In some embodiments, the length L1 of the beam 26 is between about 25% and about 75% of the length L2 of the plate body 24. In other embodiments, the length L1 of the beam 26 is between about 40% and about 60% of the length L2 of the plate body 24. The actual lengths of the beam 26 and the plate body 24 can vary depending on the fusion/fracture pattern, among other criteria.

The width W1 is defined as the distance the beam 26 extends away from the bone contacting surface 32 of the plate body 24. The beam 26 may be tapered or non-tapered in a direction of the width W1. In an embodiment, the width W1 is smaller than the length L1 of the beam 26. In some embodiments, the width W1 of the beam 26 is between about 20% to about 50% of the length L1 of the beam 26. The actual width of the beam 26 can vary depending on the fusion/fracture pattern, among other criteria.

The beam 26 may include a first beam section 46, a second beam section 48, and a bridging section 50 that connects between the first beam section 46 and the second beam section 48. The first beam section 46 and the second beam section 48 may each include the width W1. However, the bridging section 50 only extends to a width W2 (see Figure 5) from the bone contacting surface 32 of the plate body 24. In an embodiment, the width W2 is smaller than the width W1.

The beam 26 may further include a cut-out section 44 that allows for visualization of the fusion site during surgical procedures involving implantation of the internal beam plate 22. The cut-out section 44 may be flanked laterally by the first beam section 46 and the second beam section 48 and may extend up to the bridging section 50 of the beam 26. In an embodiment, the cut-out section is U-shaped. However, other shapes are contemplated within the scope of this disclosure.

The plate body 24 may include various openings 36 (e.g., threaded openings), each configured for receiving a fixation device (e.g. screw, etc., not shown in Figures 2-6) for fixating the internal beam plate 22 to one or more bones or bone segments. The openings 36 extend completely through the plate body 24 and therefore open through both the bone contacting surface 32 and the outer surface 34. In an embodiment, the first portion 28 and the second portion 30 of the plate body 24 each include a pair of openings 36. In an embodiment, the length L1 of the beam extends across a majority of a distance that spans between the pairs of openings 36 of the fist portion 28 and the second portion 30.

In an embodiment, the openings 36 of the first portion 28 are staggered along the longitudinal centerline axis A and the openings 36 of the second portion 30 are aligned along the longitudinal centerline axis A. However, other configurations are also contemplated, and thus the total number of openings 36 and their specific arrangement within the plate body 24 are not intended to limit this disclosure. The openings 36 of the first and second portions 28, 30, may be disposed outboard of the opposing lengthwise ends 40, 42 of the beam 26 (see Figure 4).

The internal beam plate 22 may be made from any biocompatible material or combinations of biocompatible materials. Exemplary materials include, but are not limited to, titanium, titanium alloys (e.g., TI-6AL4V-ELI), stainless steel, and thermoplastic materials.

In another embodiment, the internal beam plate 22 may optionally include features for achieving compression across a joint. For example, the internal beam plate 22 could include a gear-engaging mechanism (e.g., slot with integral teeth, not shown) for achieving compression across a joint.

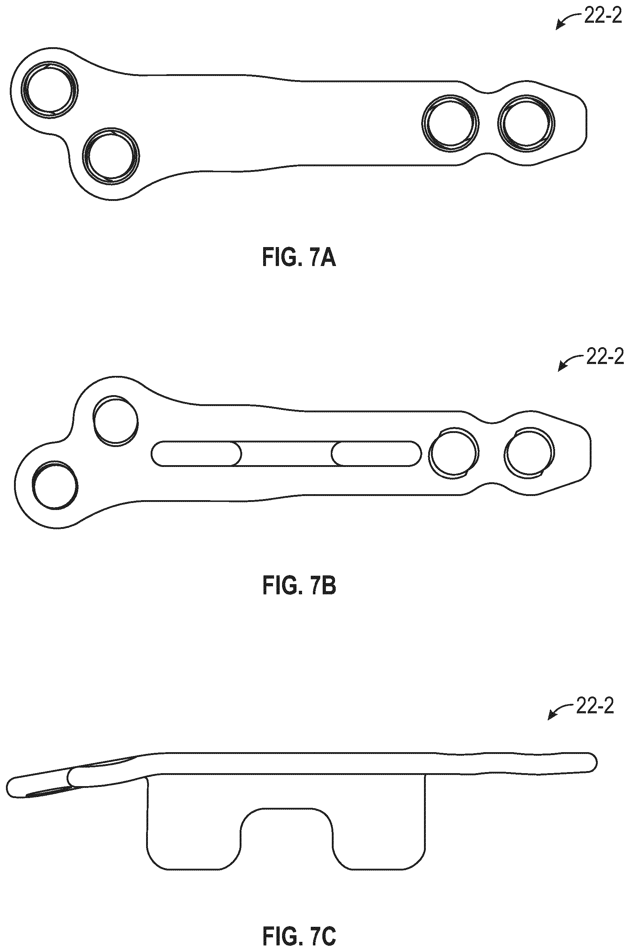

In the above embodiment, the internal beam plate 22 is shown having a right orientation that is suitable for medial placement relative to a right-side human foot. However, the internal beam plate 22 could also be provided in a left orientation, with such a bone plate being the mirror image of the right orientated bone plates described above and that is suitable for medial placement relative to a left-side human foot. Figures 7A, 7B, and 7C illustrate an exemplary internal beam plate 22-2 having a left orientation.

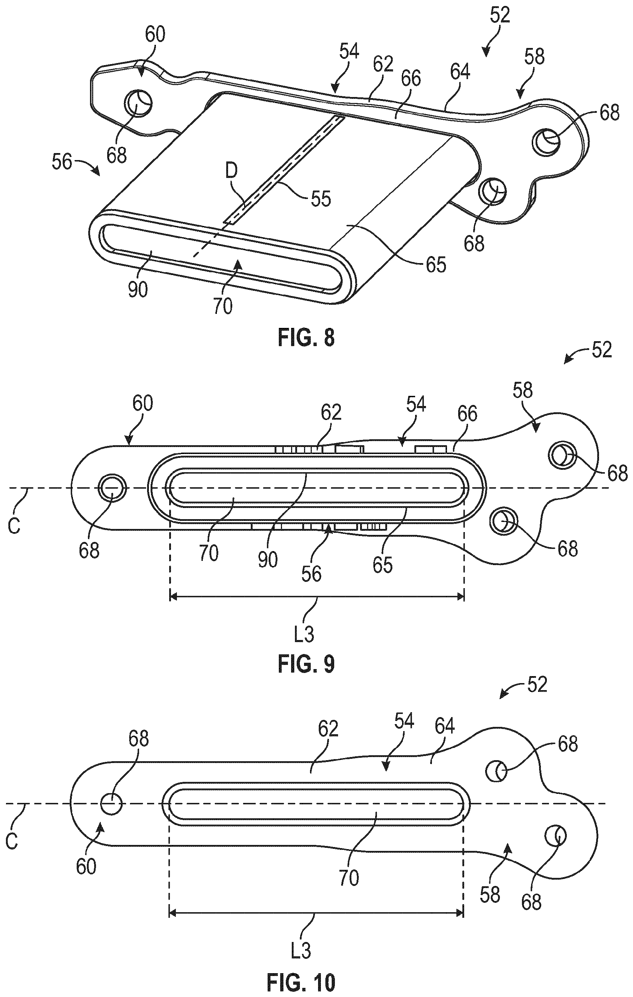

Figures 8, 9, and 10 illustrate an exemplary cutting guide 52 for preparing a bone or bones to receive the beam 26 of the internal beam plate 22 described above. The cutting guide 52 may include a base 54 and a raised housing 56 that protrudes outwardly away from the base 54. In an embodiment, the base 54 and the raised housing 56 are integrated to establish a single-piece structure. Stated another way, the cutting guide 52 may be a monolithic structure without any mechanical attachments for connecting the base 54 and the raised housing 56. In yet another embodiment, the cutting guide 52 is an additively manufactured surgical instrument.

The base 54 of the cutting guide 52 is configured to mimic the design (e.g., size, shape, etc.) of the plate body 24 of the internal beam plate 22. The base 54 may therefore extend along a longitudinal centerline axis C between a first portion 58 and a second portion 60. An intermediate portion 62 extends between the first portion 58 and the second portion 60. The base 54 additionally includes a bone contacting surface 64 and an outer surface 66 on an opposite side of the base 54 from the bone contacting surface 64.

The first portion 58 and the second portion 60 of the base 54 may each include at least one aperture 68. The apertures 68 are configured for receiving K-wires or other surgical devices for removably securing the cutting guide 52 to one or more bones during a surgical procedure. Each aperture 68 extends entirely through the thickness of the base 54 and thus opens through both the bone contacting surface 64 and the outer surface 66. In an embodiment, the first portion 58 includes two apertures 68 and the second portion 60 includes one aperture 68. However, other configurations are contemplated within the scope of this disclosure.

The raised housing 56 may protrude outwardly from the outer surface 66 of the base 54 in an opposite direction from the bone contacting surface 64 of the base 54. The raised housing 56 may protrude from the intermediate portion 62 of the base 54 and may be in the shape of an elongated ovoid. In an embodiment, the raised housing 56 protrudes away from the base 54 along an axis D that is substantially perpendicular to the longitudinal centerline axis C.

A longitudinal cutting slot 70 may be formed through the cutting guide 52. A surface 90 of the raised housing 56 may circumscribe the longitudinal cutting slot 70. The longitudinal cutting slot 70 may extend through both the base 54 and the raised housing 56 of the cutting guide 52. In an embodiment, the longitudinal cutting slot 70 extends entirely through the base 54 and the raised housing 56 and therefore opens through both the outer surface 66 and the bone contacting surface 64 of the base 54. The longitudinal cutting slot 70 may be elongated and extends in parallel with the longitudinal centerline axis C, and in the exemplary embodiment, the longitudinal centerline axis C bisects the longitudinal cutting slot 70.

The longitudinal cutting slot 70 is configured to guide a cutting tool, such as a surgical burr, for forming a slot in a bone or bones for receiving the beam 26 of the internal beam plate 22. The surface 90 of the raised housing 56 may act a hard stop for restricting the cut path of the cutting tool.

The longitudinal cutting slot 70 may include a length L3. The length L3 may be slightly larger (e.g., by about two or more mm) than the length L2 of the beam 26 of the internal beam plate 22. Oversizing the size of the slot formed by using the cutting guide 52 relative to the size of the beam 26 allows for further compression without risk of impingement between the beam 26 and the bone(s) subsequent to implantation of the internal beam plate 22.

The raised housing 56 may additionally include an alignment feature 55 (see Figure 8) that can be used for aligning the cutting guide 52 relative to a bone, bones, and/or joint. The alignment feature 55 may be formed on an exterior surface 65 of the raised housing 56 at location that coincides with a center point of the longitudinal cutting slot 70. In an embodiment, the alignment feature 55 is a rib that protrudes outwardly from the exterior surface 65. In another embodiment, the alignment features 55 is a laser line formed on the exterior surface 65. However, other configurations are contemplated within the scope of this disclosure.

The cutting guide 52 may be made from a suitable polymeric material, such as nylon 12, for example. In another embodiment, the cutting guide 52 may be made of a suitable radiolucent material. During a minimally invasive procedure when fluoroscopy is used to determine the placement of K-wires and/or other fasteners, the radiolucent cutting guide 52 that receives the K-wires will not affect the visibility of the K-wires in X-ray images. However, as would be appreciated by a person of ordinary skill in the art having the benefit of this disclosure, the cutting guide 52 could be made from any suitable medical grade material.

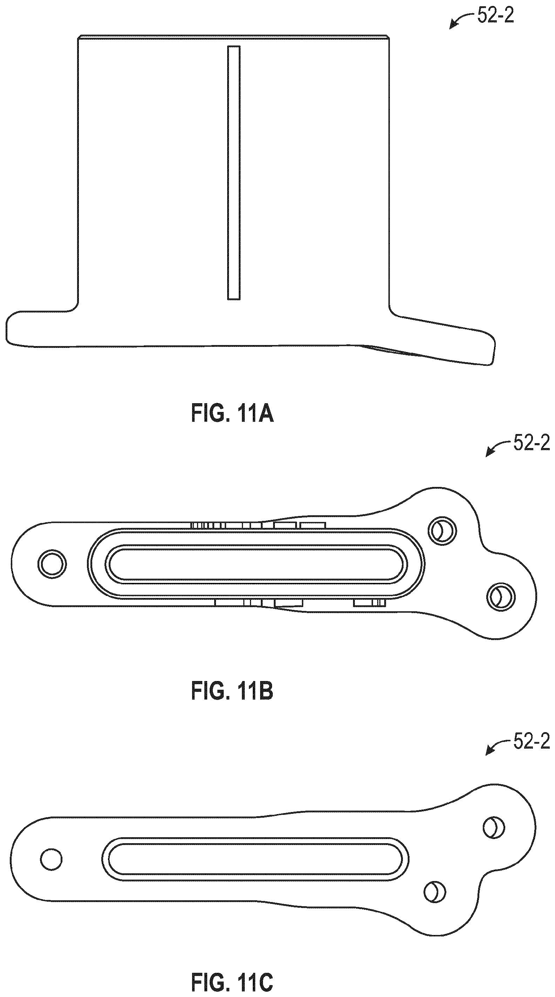

In the embodiment shown in Figures 8-10, the cutting guide 52 has a right orientation that is suitable for medial placement relative to a right-side human foot. However, the cutting guide 52 could also be provided in a left orientation, with such a guide being the mirror image of the right orientated cutting guide described above and that is suitable for medial placement relative to a left-side human foot. Figures 11A, 11B, and 11C illustrate an exemplary cutting guide 52-2 having a left orientation.

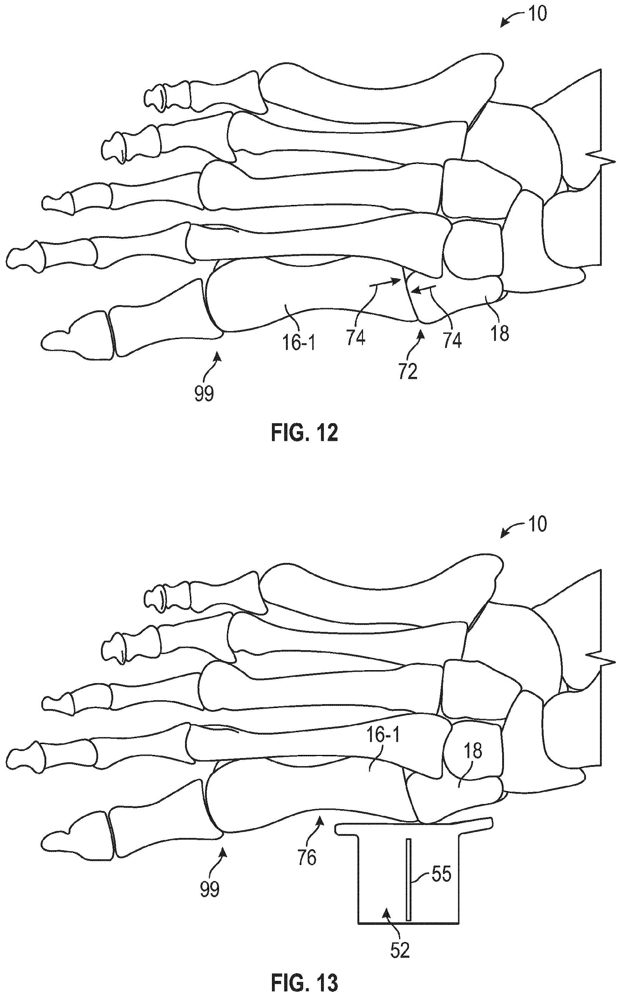

Figures 12-19, with continued reference to Figures 1-11C, schematically illustrate, in sequential order, an exemplary surgical method for performing an arthrodesis procedure, such as a Lapidus bunionectomy procedure for correcting a bunion abnormality of a first MTP joint 99 of a foot, for example. Fewer or additional steps than are recited below could be performed within the scope of this disclosure. In addition, the recited order of steps depicted in Figures 12-19 is not intended to limit this disclosure.

Referring first to Figure 12, a first tarsometatarsal (TMT) joint 72 of the foot 10 may be appropriately prepared for performing the arthrodesis procedure. Preparation may include removing articular cartilage from opposing articular surfaces 74 of the first metatarsal 16-1 and the cuneiform 18 of the first TMT joint 72. A rongeur, curette, osteotome, and/or other surgical tool may be used to remove articular cartilage and expose underlying subchondral bone at each opposing articular surface 74.

Referring next to Figure 13, the cutting guide 52 may be positioned onto the medial aspect 76 of the first TMT joint 72. The alignment feature 55 may be used to align the cutting guide 52 to a desired location or anatomical feature of the first TMT joint 72.

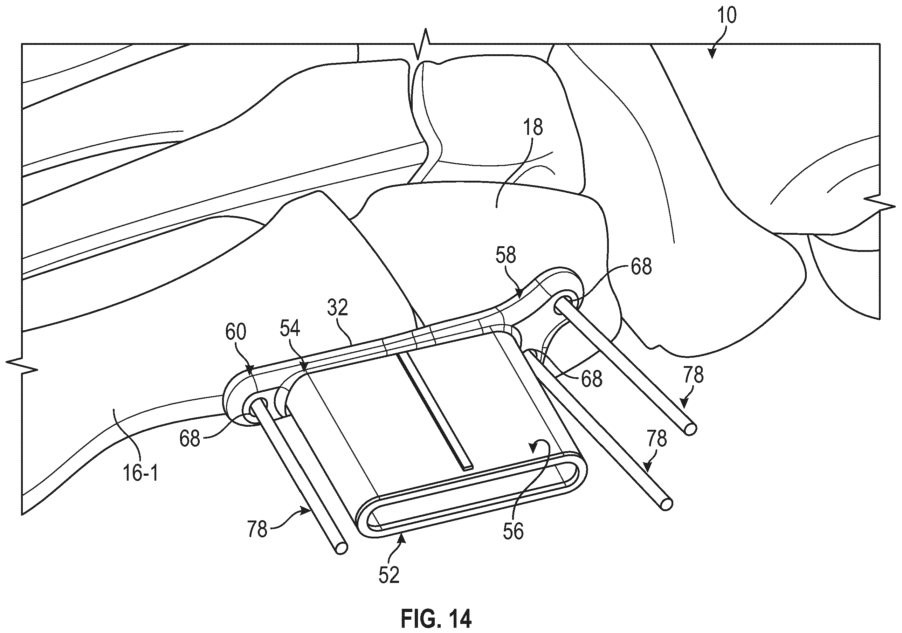

Once a desired position and alignment is achieved, the cutting guide 52 may be secured to the medial surfaces of both the first metatarsal 16-1 and the cuneiform 18 with one or more K-wires 78 (see Figure 14). One K-wire 78 may be inserted through each aperture 68 of the base 54 of the cutting guide 52 in order to temporarily fixate the cutting guide 52 in place relative to the first TMT joint 72. In the fixated position, the bone contacting surface 32 of the base 54 is positioned directly against the first metatarsal 16-1 and the cuneiform 18, the first portion 58 of the base 54 faces proximally and the second portion 60 of the base 54 faces distally.

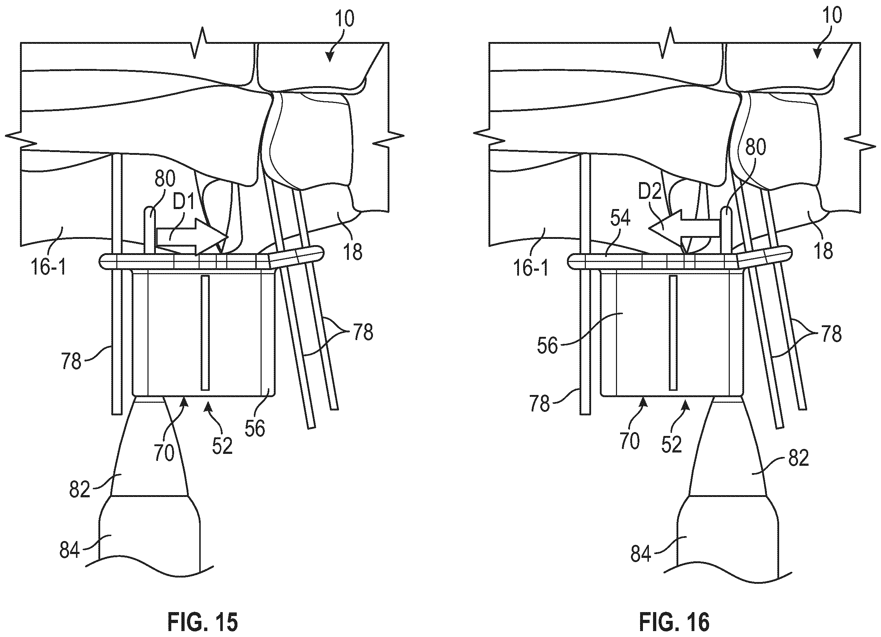

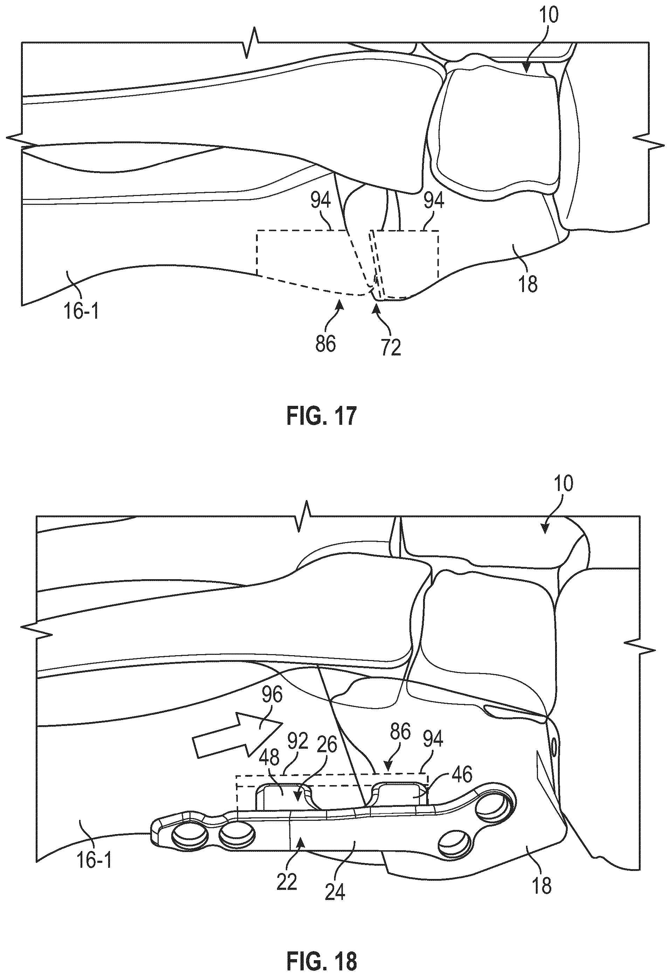

Referring next to Figures 15-17, the first metatarsal 16-1 and the cuneiform 18 may be further prepared for receiving the beam 26 of the internal beam plate 22 by using a surgical cutting device 80, such as a minimally invasive burr, for example. The surgical cutting device 80 may be connected to an adapter 82 of a powered unit 84. The surgical cutting device 80 may be inserted through the longitudinal cutting slot 70, and the powered unit 84 may then be powered on to begin drilling. The surgical cutting device 80 may be moved in a first direction D1 (e.g., to the right as schematically shown in Figure 15) and then in a second opposite direction D2 (e.g., to the left as schematically shown in Figure 16) for forming a slot 86 into the first metatarsal 16-1 and the cuneiform 18 (see Figure 17). The raised housing 56 of the cutting guide 52 delimits the cutting path of the surgical cutting device 80 as it is moved in the first direction D1 and the second direction D2.

The slot 86 may extend partially through each of the first metatarsal 16-1 and the cuneiform 18 (i.e., only extends through first cortexes of the bones), with a first portion 92 of the slot 86 formed in the first metatarsal 16-1, and a second portion 94 of the slot 86 formed in the cuneiform 18 (see Figure 17). The slot 86 is sized and shaped to accommodate the beam 26 of the internal beam plate 22. The cutting guide 52 may be removed from the first TMT joint 72 once the slot 86 is fully formed.

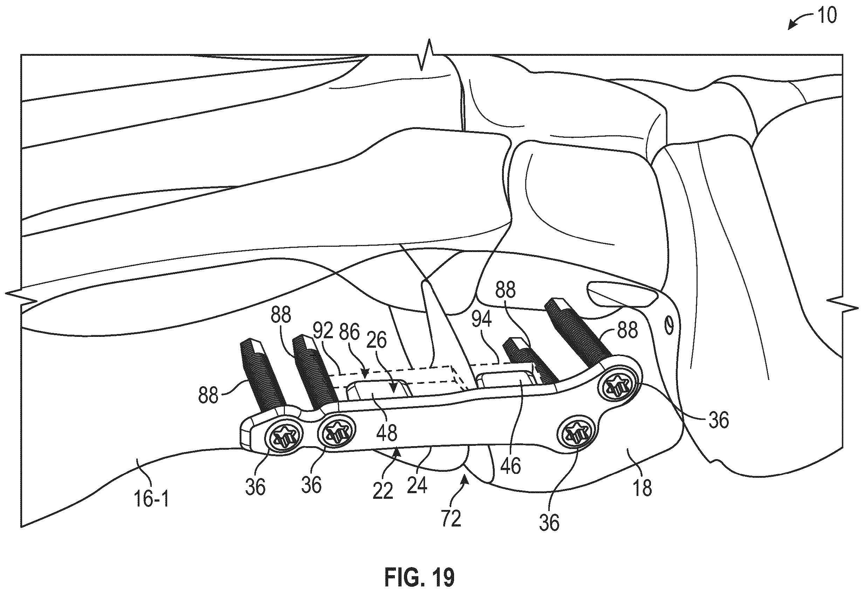

Next, as illustrated by Figures 18 and 19, the internal beam plate 22 may be positioned in place by inserting the beam 26 into the previously prepared slot 86. If necessary/desired, the first TMT joint 72 may be further compressed (see arrow 96 of Figure 18) to further reduce the first metatarsal 16-1 toward the cuneiform 18. The slightly oversized slot 86 allows for further compression without the risk of impingement between the beam 26 and either the first metatarsal 16-1 or the cuneiform 18. Once the desired intramedullary reduction is achieved, fixation devices 88, such as locking screws, for example, may be inserted into the openings 36 of the internal beam plate 22 to secure the internal beam plate 22 to the first metatarsal 16-1 and the cuneiform 18. When implanted, the first beam section 46 of the beam 26 may be accommodated within the second portion 94 of the slot 86, and the second beam section 48 may be accommodated within the first portion 92 of the slot 86. Providing the fusion at the first TMT joint 72 allows for proper function at the first MTP joint 99, thereby aiding facilitating correction of the bone abnormality 20.

The surgical instruments described herein may be packaged together and may be collectively referred to as a surgical system or a surgical kit. For example, one or more of the internal beam plates, the cutting guides, and the surgical cutting devices may be packaged together as part of a surgical system/kit.

The internal beam plates described herein are designed to increase strength and stiffness in two different planes across a fusion or fracture site with a single internal beam plate. The plates are relatively simple to implant and provide highly reproducible results. Further, the cutting guides provide for reproducible implantation of the internal beam plates.

Although the different non-limiting embodiments are illustrated as having specific components or steps, the embodiments of this disclosure are not limited to those particular combinations. It is possible to use some of the components or features from any of the non-limiting embodiments in combination with features or components from any of the other non-limiting embodiments.

It should be understood that like reference numerals identify corresponding or similar elements throughout the several drawings. It should further be understood that although a particular component arrangement is disclosed and illustrated in these exemplary embodiments, other arrangements could also benefit from the teachings of this disclosure.

The foregoing description shall be interpreted as illustrative and not in any limiting sense. A worker of ordinary skill in the art would understand that certain modifications could come within the scope of this disclosure. For these reasons, the following claims should be studied to determine the true scope and content of this disclosure.

## Claims

1. An internal beam plate, comprising:
a plate body extending along a longitudinal centerline axis between a first portion and a second portion and including a bone contacting surface and an outer surface opposed to the bone contacting surface;
a beam that protrudes outwardly from the bone contacting surface; and
a cut-out section formed in the beam.

2. The internal beam plate as recited in claim 1, wherein the bone contacting surface includes a curvature for conforming to a contour of a bone.

3. The internal beam plate as recited in claim 1 or 2, wherein the plate body and the beam are integrated to establish a single-piece structure that excludes mechanical attachments for connecting the plate body and the beam together.

4. The internal beam plate as recited in any preceding claim, wherein the beam protrudes away from the bone contacting surface along a beam axis that is substantially perpendicular to the longitudinal centerline axis.

5. The internal beam plate as recited in claim 4, wherein a distance the beam protrudes from the bone contacting surface is smaller than a first length of the beam and a second length of the plate body.

6. The internal beam plate as recited in claim 5, wherein the first length is less than the second length.

7. The internal beam plate as recited in any preceding claim, comprising a first set of openings formed through the first portion of the plate body and a second set of openings formed through the second portion of the plate body.

8. The internal beam plate as recited in any preceding claim, wherein the cut-out section is flanked by a first beam section and a second beam section of the beam.

9. The internal beam plate as recited in claim 8, wherein a bridging section of the beam connects between the first beam section and the second beam section.

10. The internal beam plate as recited in claim 9, wherein the first beam section and the second beam section each extend a first distance from the bone contacting surface of the plate body, and the bridging section extends a second, lesser distance from the bone contacting surface of the plate body.

11. A cutting guide for preparing a joint for a surgical procedure, comprising:
a base extending along a longitudinal centerline axis between a first portion and a second portion and including a bone contacting surface and an outer surface opposed to the bone contacting surface;
a raised housing protruding outward from the outer surface of the base; and
a longitudinal cutting slot formed through the raised housing and the base and extending along the longitudinal centerline axis.

12. The cutting guide as recited in claim 11, wherein the base includes an intermediate portion that connects between the first portion and the second portion, and further wherein the raised housing protrudes outwardly from the outer surface at the intermediate portion.

13. The cutting guide as recited in claim 11 or 12, comprising an alignment feature provided at an exterior surface of the raised housing.

14. The cutting guide as recited in any one of claims 11 to 13, comprising at least one aperture formed through each of the first portion and the second portion of the base.

15. The cutting guide as recited in any one of claims 11 to 14, wherein the raised housing is ovoid-shaped.
